# EUROPEAN PATENT SPECIFICATION

(11) **EP 1 071 480 B1**
(45) Date of publication and mention of the grant of the patent: **10.11.2010**
(21) Application number: 99909646.4
(22) Date of filing: 25.02.1999
(51) Int. Cl.: A61L 17/00

(54) **Sutures made from absorbable copolymers**
Chirurgisches Nahtmaterial hergestellt aus resorbierbaren Copolymeren
Sutures faites de copolymers absorbable

(30) Priority: 25.02.1998 US 30191; 26.09.1998 US 161606
(43) Date of publication of application: 31.01.2001
(73) Proprietor: Tyco Healthcare Group LP, North Haven, CT 06473 (US)
(72) Inventor: ROBY, Mark, S., Killingworth, CT 06419 (US); JIENG, Ying, Raleigh, NC 27615 (US); KOKISH, Lyudmila, K., Orange, CT 06477 (US)
(74) Representative: HOFFMANN EITLE
(86) International application number: PCT/US1999/004242
(87) International publication number: WO 1999/043364

(56) References cited:
- EP-A- 0 626 404
- EP-A- 0 635 274
- EP-A- 0 761 712
- US-A- 3 092 891
- US-A- 4 700 704
- US-A- 5 133 739
- US-A- 5 431 679
- US-A- 5 718 716

## Description

### TECHNICAL FIELD

Absorbable copolymers of randomly polymerized glycolide and caprolactone are described. Processes for making the copolymers and surgical articles made totally or in part from such copolymers, including sutures, are also described.

### BACKGROUND

Bioabsorbable surgical devices made from copolymers derived from glycolide and epsilon-caprolactone are known in the art. Such bioabsorbable surgical devices include surgical sutures.

A desirable characteristic of a bioabsorbable suture is its ability to exhibit and maintain desired tensile properties for a predetermined time period followed by rapid absorption of the suture mass (hereinafter "mass loss".)

Synthetic absorbable sutures are known in the art. Absorbable multifilament sutures such as DEXON sutures (made from glycolide homopolymer and commercially available from Davis & Geck, Danbury, Connecticut), VICRYL sutures (made from a copolymer of glycolide and lactide and commercially available from Ethicon, Inc., Sommerville, New Jersey), and POLYSORB sutures (also made from a copolymer of glycolide and lactide and commercially available from United States Surgical Corporation, Norwalk, Connecticut) are known in the industry as short term absorbable sutures. The classification short term absorbable sutures generally refers to surgical sutures which retain at least about 20 percent of their original strength at three weeks after implantation, with the suture mass being essentially absorbed in the body within about 60 to 90 days post implantation.

Long term absorbable sutures are generally classified as sutures capable of retaining at least about 20 percent of their original strength for six or more weeks after implantation, with the suture mass being essentially absorbed in the body within about 180 days post implantation. For example, PDS II sutures (commercially available from Ethicon, Inc., Sommerville, New Jersey), are synthetic absorbable monofilament sutures that reportedly retain at least about 20 to 30 percent of its original strength six weeks after implantation. However, PDS II reportedly exhibits minimal mass loss until 90 days after implantation with the suture mass being essentially absorbed in the body about 180 days after implantation. MAXON suture (commercially available from Davis & Geck, Danbury, Connecticut) is another absorbable synthetic monofilament that reportedly generally fits this absorption profile.

Most recently, United States Surgical Corporation has introduced BIOSYN monofilament sutures which exhibit good flexibility, handling characteristics, knot strength and absorption characteristics similar to those of presently available short term absorbable multifilament sutures.

Another attempt to provide an acceptable synthetic absorbable monofilament sutures resulted in MONOCRYL, a suture fabricated from an absorbable block copolymer contain glycolide and caprolactone, commercially available from Ethicon, Inc..

However, no synthetic absorbable monofilament sutures exist today which approximate the strength retention, mass loss, and modulus of sutures commonly referred to in the art as "catgut" or, "gut" sutures. It is well known in the art that the term gut suture refers to a collagen based suture of any type or origin often fabricated from the mammalian intestines, such as the serosal layer of bovine intestines or the submucosal fibrous layer of layer sheep intestines. Gut sutures exhibit the unique combination of two week strength retention and about 75 day mass loss while maintaining acceptable modulus and tensile strength; and thus are still wisely used in gynecological surgery.

It would be advantageous to provide a synthetic absorbable suture which exhibits physical properties similar to the gut suture.

U.S. Patent No. 4.700,704 to Jamiolkowski does teach that sutures can be fabricated from random copolymers of glycolide and epsilon-caprolactone, and more specifically from random copolymers containing from 20 to 35 weight percent epsilon-caprolactone and from 65 to 80 weight percent glycolide. Moreover, Jamiolkowski reports that sutures fabricated from glycolide/epsilon-caprolactone copolymers containing over 35% caprolactone under are not orientable to a dimensionally stable fiber. Jamiolkowski further reports that some sutures fabricated from glycolide/epsilon-caprolactone copolymers containing 15% caprolactone are also not orientable to a dimensionally stable fiber. Furhermore, Jamiolkowski also reports the undesirable combination of low modulus and low tensile strength for the glycolide/ epsilon-caprolactone copolymers which he was able to fabricate into sutures.

Therefore, it would be unexpected that sutures made from random copolymer of glycolide and epsilon-caprolactone would provide the strength retention and mass loss characteristics approximating those of gut sutures while maintaining an acceptable modulus and tensile strength.

### SUMMARY

It has now surprisingly been found that a suture formed from a random copolymer of glycolide and caprolactone exhibits strength retention, mass loss and modulus similar to that of gut sutures. The copolymers used in forking surgical articles include between 25 and 32 weight percent or hydroxy caproic acid ester units and between 75 and 68 weight percent of glycolic acid ester units.

The random copolymers can be spun into fibers. The fibers can be advantageously fabricated into either monofilament or multifilament sutures having physical properties, similar to those of gut sutures.

In addition, a process of making such synthetic absorbable monofilament sutures from the above described caprolactone/glycolide random copolymers has been found. The process, for a given size suture, comprises the operations of extruding the random caprolactone/glycolide copolymer at an extrusion temperature, of from 70°C to 215°C to provide a monofilament fiber, passing the solidified monofilament through water (or other suitable liquid medium) quench bath at a temperature of from 15° C to 25° C or through in air (or other suitable gaseous medium) at from 15°C to 25°C, stretching the monofilament through a series air ovens at an overall stretch ratio of from 7:1 to 14:1 to provide a stretched monofilament. In a particularly useful embodiment, the monofilament is stretched through three air ovens by four godet stations. The first air oven is maintained at ambient temperature, whereas the second air oven is heated to a temperature above the crystalization temperature of the glycolide /epsilon caprolactone copolymer at 80° C to 110° C, and the third air oven is set at 85° C to 120° C. The draw ratio between the first and second godet station ranges between 5:1 to 8:1. The draw ratio between the second and third godet station ranges between 1.3:1 to 1.8:1. The draw ratio between the third and fourth godet station ranges between 1.04:1 to 1.06:1. The sutures then may be annealed with or without relaxation at a temperature of from 80°C to 120°C to provide the finished suture.
Fig. 1 is a schematic illustration of an apparatus which is suitable for manufacturing of monofilament sutures disclosed herein; and
Fig. 2 is a perspective view of a suture attached to a needle.
Figs. 3A-3C illustrate the formation of the knot which was employed in in the loop pull test used in Example 2.

### DESCRIPTION OF THE PREFERRED EMBODIMENTS

It has been found that glycolide and epsiloncaprolactone monomers can advantageously be combined to form a random copolymer useful in forming sutures having strength retention, mass loss, and modulus characteristics similar to or superior to gut sutures.

The random copolymer can be prepared using conventional techniques. For example, monomers can be dried, mixed in a reaction vessel with an initiator (either a single or multifunctional initiator) and a suitable polymerization catalyst and heated at temperatures from 170°C to 200°C for a period of time ranging from 10 hours to 30 hours.

The copolymer has repeating units derived from glycolide randomly combined with repeating units derived from caprolactone. Repealing units derived from glycolide, comprise between 25 and 32 weight percent of the copolymer and preferably 30 weight, percent of caprolactone and 70 weight percent of gylcolide. Copolymers of caprolactone and glycolide having an inherent viscosity of from 1.0 to 1.8 dl/g measured at 30°C and at a concentration of 0.25 g/dl in chloroform or HFIP may generally be used.

The random copolymers can be formed into sutures using any known technique, such as, for example, extrusion, molding and/or solvent casting. The copolymer can be used alone, blended with other absorbable compositions, or in combination with non-absorbable components. Sutures can be manufactured from the copolymers described herein. The copolymers are spun into fibers to be used in making sutures.

Multifilament sutures of the present invention may be made by methods known in the art. Braid constructions such as those disclosed and claimed in U.S. Patent No. s 5,059,213 and 5,019,093 are suitable for the multifilament suture of the present invention.

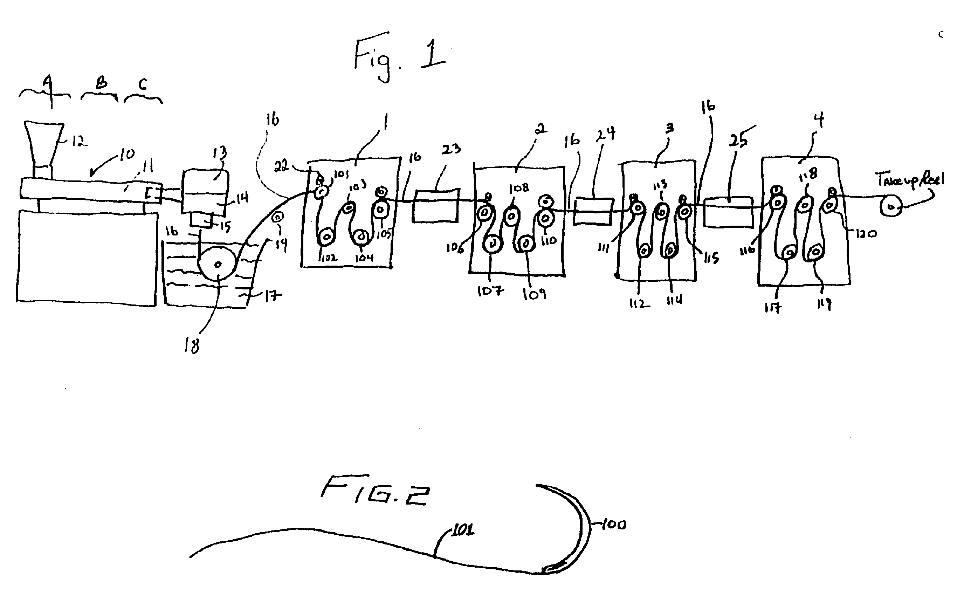

Fig. 1 substantially illustrated the extruding, quenching and stretching operations of the monofilament manufacturing operation herein. Extruder unit 10 is of a known or conventional type and is equipped with controls for regulating the temperature of barrel 11 in various zones thereof, e.g., progressively higher temperatures in three consecutive zones A, B and C along the length of the barrel. Pellets or powder of resins of the present invention are introduced to the extruder through hopper 12. Any of the above described copolymers which are useful for the formation of fibers can be used herein.

Motor-driven metering pump 13 delivers melt extruded resin at a constant rate to spin pack 14 and hereafter through spinneret 15 possessing one or more orifices of desired diameter to provide a molten monofilament 16 which then enters quench bath 17, e.g., containing water, where the monofilament solidifies. The distance monofilament 16 travels after emerging from spinneret 15 to the point where it enters quench bath 17, i.e., the air gap, can vary and can advantageously be from 0.5 to 100 cm and preferably from 1 to 20 cm. If desired, a chimney (not shown), or shield, can be provided to isolate monofilament 16 from contact with air currents which might otherwise affect the cooling of the monofilament in an unpredictable manner. In general, barrel zone A of the extruder can be maintained at a temperature of from 170°C to 215°C, zone B at from 1.70°C to 215°C and zone C at from 170°C to 215°C. Additional temperature parameters include: metering pump block 13 at from 170°C to 215°C, spinneret 15 at from 170°C to 225°C and quench bath at from 15°C to 40°C.

Monofilament 16 is passed through quench bath 17 around driven roller 18 and over idle roller 19. Optionally, a wiper (not shown) may remove excess water from the monofilament as it is removed from quench bath 17. On existing the quench bath the monofilament is passed through first godet station 1, which is equiped with five individual godets, i.e. godets 101, 102, 103, 104 and 105. Upon entering godet station 1, monofilament 16 is wrapped around a first godet 101 provided with nip roll 22 to prevent slippage which might otherwise result from the subsequent stretching operation; and subsequently passed over godet 101, under godet 102, over godet 103, under godet 104, and over godet 105 to godet station 2, containing godets 106, 107, 108, 109, and 110, where it is wrapped over godet 106, under godets 107, over godet 108, under godet 109, and over godet 110.. Monofilament 16 passing from godet station 1 to godet station 2 is drawn through air oven 23 at a temperature ranging from 20°C to 30°C by the godets of godet station 2 which rotate at speeds faster than the speed of the godet station 1 to provide the desired draw ratio, which is from 5:1 to 10:1 and preferably from 6:1 to 8:1, to effect the molecular orientation of the copolymer from which it is fabricated and thereby increase its tensile strength.

Following the initial draw at ambient temperature, monofilament 16 is then subjected to a second and a third drawing operation. Monofilament 16 is subsequently drawn from godet 105 through air oven 24, which is maintained at from 80°C to 110°C, to godet station 3 containing godets 111, 112, 113, 114, and 115 where it is wrapped over godet 111, under godet 112, over godet 113, under godet 114, and over godet 115. Godet station 3 spins faster than godet: station 2 to provide the desired draw ratio, which is from 1.3:1 to 1.8:1. Monofilament 16 is then drawn from godet 115 through air oven 25, which is maintained at from 85°C to 120°C, by godet station 4, containing godets 116, 117 118, 119, and 120 where it is wrapped over godet 116, under godet 117, over godet 118, under godet 119, and over godet 120. Godet station 4 spins faster than godet station 3 to provide the desired draw ratio, which is from 1.05:1 to 1.06:1. It should be understood that the godet arrangements in each of godet stations 1, 2, 3, and 4, respectively should not be limited to the above described arrangement and that each godet station may have any suitable godet arrangement.

In an alternative operation for sutures for smaller size sutures, sizes 4/0 to 8/0, monofilament 16 is only passed through godet stations 1 and 2 and not subjected to any further stetching operations.

Annealing of the suture also may be accomplished with or without shrinkage of the suture. In carrying out the annealing operation, the desired length of suture may be wound around a creel and the creel placed in a heating cabinet under nitrogen flow maintained at the desired temperature, e.g. 70°C to 120°C, as described in U.S. Patent No. 3,630,205. After a suitable period of residency in the heating cabinet. e.g., for up to about 18 hours or so, the suture will have undergone essentially no shrinkage. As shown in U.S. Patent No. 3,630,205, the creel may be rotated within the heating cabinet in order to insure uniform heating of the monofilament or the cabinet may be of the circulating hot air type in which case uniform heating of the monofilament will be achieved without the need to rotate the creel. Thereafter, the creel with its annealed suture is removed from the heating cabinet and when returned to room temperature, the suture is removed from the creel, conveniently by cutting the wound monofilament at opposite ends of the creel. The annealed sutures, optionally attached to surgical needles, are then ready to be package and sterilised.

Alternatively, the suture may be annealed on line with or without relaxation. For relaxation, the fourth godet station rotates at a slower speed than the third godet station thus relieving tension on the filament.

The suture disclosed herein, suture 101, may be attached to a surgical needle 100 as shown in Fig. 2 by methods well known in the art. Wounds may be sutured by parsing the needled suture through tissue to create wound cloture. The needle preferably is then removed from the suture and the suture tied.

It is further within the scope of this invention to incorporate one or more medico-surgically useful substances into the present invention, e.g., those which accelerate or beneficially modify the healing process when particles are applied to a surgical repair site. So, for example, the suture can carry a therapeutic agent which will be deposited at the repair site. The therapeutic agent can be chosen for its antimicrobial properties, capability for promoting repair or reconstruction and/or new tissue growth. Antimicrobial agents such as broad spectrum antibiotic (gentamycin sulfate, erythromycin or derivatized glycopeptides) which are slowly released into the tissue can be applied in this manner to aid in combating clinical and sub-clinical infections in a tissue repair site. To promote repair and/or tissue growth, one or several growth promoting factors can be introduced into the sutures, e.g., fibroblast growth factor, bone growth factor, epidermal growth factor, platelet derived growth factor, macrophage derived growth factor, alveolar derived growth factor, monocyte derived growth factor, magainin, and so forth, Some therapeutic indications are: glycerol with tissue or kidney plasminogen activator to cause thrombosis, superoxide dimutase to scavenge tissue damaging free radicals, tumor necrosis factor for cancer therapy or colony stimulating factor and interferon, interleukin-2 or other lymphokine to enhance the immune system.

It is contemplated that it may be desirable to dye the sutures of the present invention in order to increase visibility of the suture in the surgical field. Dyes known to be suitable for incorporation in sutures can be used. Such dyes include but are not limited to carbon black, bone black, D&C Green No. 6, and D&C Violet No. 2 as described in the handbook of U.S. colorants for Food, Drugs and Cosmetics by Daniel M. Marrion (1979). Preferably, sutures in accordance with the invention are dyed by adding up to about a few percent and preferably 0.2% dye, such as D&C Violet No. 2 to the resin prior to extrusion.

In order that those skilled in the art may be better able to practice the compositions and methods described herein, the following example is given as an illustration of the preparation of random copolymers as well as of the preparation and superior characteristics of sutures made from the random copolymers. It should be noted that the invention is not limited to the specific details embodied in the examples and further that all ratios or parts recited are by weight, unless otherwise indicated.

### EXAMPLE 1

Dry glycolide (4200 grams) and undistilled epsilon-caprolactone were added to a reactor along with 0.35 grams of distillled stannous octoate and 3 grams of 1,6 hexanediol. The mixture was dried for about 48 hours with agitation under flow of nitrogen. The reactor temperature was then set at 100°C. When the temperature of the reactants reached 100°C the temperature was maintained for about 15 minutes at which point the temperature of the reactants was raised to about 150°C and the reaction vessel heated for about an additional. 15 minutes. The temperature of the reactants was then raised to about 190°C and polymerization conducted with stirring under a nitrogen atmosphere for about 18 hours. The reaction product is then isolated, comminuted, and treated to remove residual reactants using known techniques. The treatment to remove residual reactants occurs at 130°C for 48 hours under vaccuum.

Table I below sets forth typical conditions for extruding, stretching of size 3/0 sutures in accordance with this invention. All of the monofilament sutures were fabricated from the resin of Example 1.

**TABLE I**

| CONDITIONS OF MANUFACTURING VARIOUS SIZES OF MONOFILAMENT OF THE PRESENT INVENTION | |
|---|---|
| Example | 1 |
| Suture Size | 3/0 |
| Process Conditions | Extrusion |
| extruder screw, rpm | 7 |
| pump, rpm | 15.4 |
| driven roller, mpm | 2.7 |
| barrel temp., °C. zone A | 183 |
| barrel temp., °C, zone B | 186 |
| barrel temp., °C, zone C | 189 |
| clamp temp., °C, | 188 |
| adapter temp., °C | 189 |
| pump temp., °C | 196 |
| block temp., °C | 190 |
| barrel melt temp., °C | 192 |
| pump melt temp., °C | 191 |
| spinneret melt temp., °C | 194 |
| barrel pressure, psi | 1040 |
| pump pressure, psi | 1000 |
| spinneret pressure, psi | 1400 |
| pump size, cc per revolution | 0.16 |
| diameter of spinneret, orifices, mm | 1.2 |
| no. of spinneret orifices | 1 |
| quench bath temp., °C | 20 |
| Stretching (Orienting) Operation | |
| Example | |
| draw bath temp., °C | ambient |
| first godet station, mpm | 2.9 |
| second godet, mpm | 20.8 |
| third godet station, mpm | 34.6 |
| fourth godet station,mpm | 36.2 |
| first oven temp, °C | 28 |
| second oven temp, °C | 85 |
| third oven temp, °C | 90 |
| | |
| overall draw ratio | 12.57:1 |

### Annealing Operation

| Example | 1 |
|---|---|
| annealing temp., °C | 80°C |
| time (hrs.) | 6 |

The physical properties of then sutures and the procedures employed for their measurement are set forth in Table II as follows:

**TABLE II**

| PROCEDURES FOR MEASURING PHYSICAL PROPERTIES OF MONOFILAMENT SUTURES OF THE PRESENT INVENTION | |
|---|---|
| Physical Property | Test: Procedure |
| knot-pull strength, kg | U.S.P. XXI, tensile strength, sutures (881) |
| straight-pull strength, kg | ASTM D-2256, Instron Corporation |
| elongation, % | ASTM D-2256 |
| tensile strength, kg/mm² | ASTM D-2256, Instron Corporation Series IX Automated Materials Testing System 1.03A |
| Young's Modulus | Instron Merlin Software version 2000 Series IX calculation 18.3 (commercially available from Instron Corporation) |

Table III below sets forth the physical properties of the size 3/0 suture of the present invention.

**TABLE III**

| Physical Property | Example 1 |
|---|---|
| diameter (mm) | .298 |
| knot-pull strength (kg) | 2.66 |
| Young's Modulus (kpsi) | 170 |
| Elongation % | 22 |
| Tensile Strength (kpsi) | 102.2 |

As the data in Tables III illustrates, the suture made of the copolymer provided herein shows a desired physical properties, such as modulus and tensile strength.

### Example 2

### INVITRO STRENGTH RETENTION

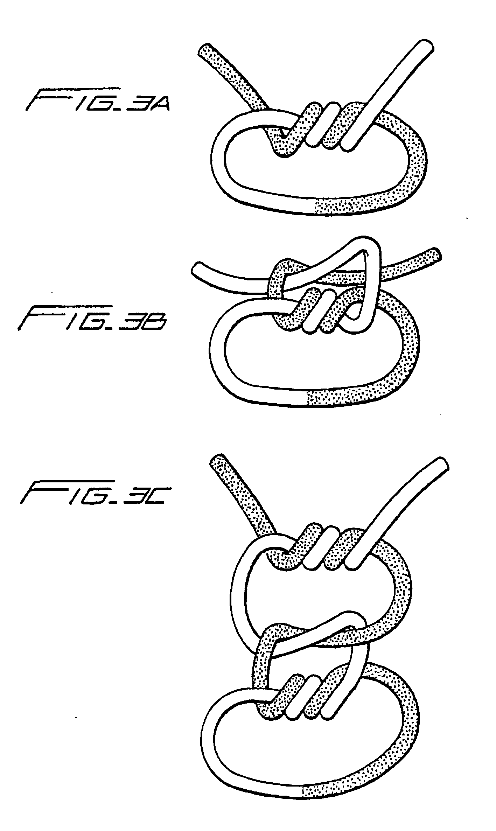

Monofilament sutures manufactured in accordance with the above described process using the copolymer of Example 1 were tested for in vitro strength retention. In vitro loop-pull strength retention is indicative of in vivo strength retention. The in vitro strength retention of the suture was tested as follows:
To simulate in vivo conditions, the suture samples were stored in a container filled with Sorenson's buffer solution at 37°C. After various periods of time, the suture samples were then removed from the container to test their loop-pull strength as follows. A knotted loop was formed in a test suture in three steps as shown in FIGS. 3A - 3C. As shown in step 1 of of ETG 3A, each suture was given a double throw (left over right ) around a 2 cm diameter cylinder. In Step 2, the free ends of the suture were set in a single throw throw (right over left) onto the initial throw of step 1. Finally, in step 3, another double throw ( left over right) was set onto the single throw of Step 2 to complete the knot. The free ends of the suture were cut to approximately 0.5 inches and the loop was carefully eased from the cylinder.

Testing of the loop was carried out useing an Instron Corporation (Canton, Mass.) Tensile Tester Model No. 4307, operated with a crosshead speed of 51 mm/min and equipped with flat grips, each having a pin over which the loop is positioned.

The results of the tests are presented in Table IV hereinbelow. In the strength retention data reported in Table II, Tₙ represents the time elapsed in weeks since the sample was placed in the solution, with n representing the number of weeks.

**TABLE IV**

| | PERCENTAGE OF IN VITRO STRENGHT RETAINED | | | | | | | |
|---|---|---|---|---|---|---|---|---|
| COMPOSITION | T₁ | T₂ | T₃ | T₄ | T₆ | T₈ | T₁₀ | T₁₂ |
| EXAMPLE I | 44 | 11 | 0 | -- | -- | -- | -- | -- |

### EXAMPLE 3

### IN VITRO MASS LOSS

Monofilament sutures manufactured in accordance with the above described process using the copolymer of Example 1 were tested for in vitro mass retention. In vitro mass retention strength is indicative of in vivo mass retention. The in vitro strength retention of the suture was tested as follows:
To simulate in vivo conditions, the suture samples were stored in a container filled with Sorenson's buffer solution at 80°C. After various periods of time, the suture samples were then removed from the container filterred, rinsed with distiioled water and dried for about 6 hours at about 40°C under vaccum and subsequently weighed.

The results of the tests are presented in Table V hereinbelow. In the strength retention data reported in Table V, Tₙ represents the time elapsed in hours since the sample was placed in the solution, with n representing the number of hours. It is well known in the art that one hour of immersion in the the container filled with Sorenson's buffer solution at 80°C approximates about one week of invivo mass loss. For comparison purposes, the same tests were conducted on Monocryl sutures.

All comparative tests were performed on size 3/0 sutures.

**TABLE V**

| | PERCENTAGE OF IN VITRO MASS RETAINED | | | | | | | |
|---|---|---|---|---|---|---|---|---|
| COMPOSITION | T₁ | T₂ | T₃ | T₄ | T₆ | T₈ | T₁₀ | T₁₂ |
| EXAMPLE I | 92.79 | 66.35 | 51 | 37.73 | 34.31 | 29.35 | 26.97 | 23.58 |
| Monocryl | 94.86 | 74.79 | 66.83 | 47.95 | 42.63 | 35.31 | 32.14 | 27.32 |

## Claims

1. A suture fabricated from a random copolymer comprising from 68 to 75 weight percent glycolide and 25 to 32 weight percent epsilon-caprolactone, the suture exhibiting two week strength retention, mass loss of about 50% in 32 hours as measured in Sorenson's buffer solution at 80 °C and a Young's modulus ranging from 150 kpsi to 250 kpsi and a knot pull strength of 1.7 to 2.8 kg.

2. The suture of claim 1, wherein the random copolymer comprises 30 weight percent epsilon-caprolactone and 70 weight percent glycolide.

3. The suture of claim 1, wherein the suture is a size 3/0 suture and the modulus is 170 kpsi.

4. The suture of claim 1, wherein the suture is a size 3/0 suture and the knot pull strength is 2.6 kg.

5. The suture of claim 1, wherein the suture is a size 3/0 suture and the tensile strength is 102 kpsi.

6. The suture of claim 1, wherein the suture is a size 3/0 suture and exhibits the following characteristics:
| | |
|---|---|
| modulus | 170 kpsi |
| knot pull strength | 2.6 kg |
| tensile strength | 102 kpsi. |

7. The suture of claim 1 comprising a medico-surgically useful substance.

8. The suture of claim 1, wherein the random copolymer possesses an inherent viscosity of 1.0 to 1.8 dl/g at 30 °C and a concentration of 0.25 g/dl in HFIP.

9. The suture of claim 1, wherein the suture is a size 3/0 suture and exhibits a mass loss of 50% after 32 hours in Sorenson's buffer solution at 80 °C.

10. The suture of claim 1, wherein the suture is a size 3/0 suture and exhibits a mass loss of 30% after 72 hours in Sorenson's buffer solution at 80 °C.

11. The suture of claim 1, wherein the suture is a size 3/0 suture and exhibits a mass loss of 12% after 120 hours in Sorenson's buffer solution at 80 °C.

12. A process for manufacturing a monofilament suture as defined in claim 1 from a resin of a random copolymer, the random copolymer comprising epsilon-caprolactone and glycolide, which comprises the operations of:
a. extruding said resin at an extrusion temperature of from 70 °C to 215 °C to provide a monofilament;
b. stretching the solidified monofilament at a stretch ratio of from 7:1 to 14:1 to provide a stretched monofilament.

13. The process of claim 12 further comprising the steps of:
a. annealing said stretched monofilament at a temperature of from 80°C to 180 °C to provide a finished suture.

14. A method for manufacturing a monofilament suture as defined in claim 1 from a resin of a random copolymer, the random copolymer comprising epsilon-caprolactone and glycolide, which comprises:
a) extruding the copolymer to provide a molten monofilament;
b) quenching the molten monofilament to provide a solidified monofilament;
c) drawing the solidified monofilament through an air oven maintained at a temperature of 20 °C to 30 °C at a draw ratio of 5:1 to 10:1;
d) drawing the monofilament through an air oven maintained at a temperature of 80 °C to 110 °C at a draw ratio of 1.5:1 to 1.8:1;
e) drawing the monofilament through an air oven maintained at a temperature of 85 °C to 120 °C at a draw ratio of 1.05:1 to 1.06:1; and
f) annealing the monofilament.

15. The method of claim 14, wherein the random copolymer contains 30 weight percent epsilon-caprolactone and 70 weight percent glycolide.

## Patentansprüche

1. Faden, der aus einem zufälligen Copolymer gebildet wird, umfassend zwischen 68 und 75 Gew.-% Glycolide und 25 bis 32 Gew.-% Epsilon-Caprolactone, wobei der Faden zwei Wochen Festigkeitslaufzeit, einen Massenverlust von etwa 50% in 32 Stunden, gemessen in Sorenson's Pufferlösung bei 80°C, und einen Young Modulus im Bereich von 150 kpsi bis 250 kpsi und eine Knotenzugfestigkeit von 1,7 bis 2,8 kg aufzeigt.

2. Faden nach Anspruch 1, wobei das zufällige Copolymer 30 Gew.-% Epsilon-Caprolactone und 70 Gew.-% Glycolide aufweist.

3. Faden nach Anspruch 1, wobei der Faden ein Faden der Größe 3/0 und der Modulus 170 kpsi ist.

4. Faden nach Anspruch 1, wobei der Faden ein Faden der Größe 3/0 und die Knotenzugfestigkeit 2,6 kg ist.

5. Faden nach Anspruch 1, wobei der Faden ein Faden der Größe 3/0 und die Zugfestigkeit 102 kpsi ist.

6. Faden nach Anspruch 1, wobei der Faden ein Faden der Größe 3/0 ist und die folgenden Charakteristika aufweist:
| | |
|---|---|
| Modulus | 170 kpsi |
| Knotenzugfestigkeit | 2,6 kg |
| Zugfestigkeit | 102 kpsi. |

7. Faden nach Anspruch 1, umfassend eine medizinischchirurgisch nützliche Substanz.

8. Faden nach Anspruch 1, wobei das zufällige Copolymer eine inhärente Viskosität von 1,0 bis 1,8 dl/g bei 30°C und eine Konzentration von 0,25 g/dl in HFIP aufweist.

9. Faden nach Anspruch 1, wobei der Faden ein Faden der Größe 3/0 ist und einen Massenverlust von 50% nach 32 Stunden in Sorenson's Pufferlösung bei 80°C aufzeigt.

10. Faden nach Anspruch 1, wobei der Faden ein Faden der Größe 3/0 ist und einen Massenverlust von 30% nach 72 Stunden in Sorenson's Pufferlösung bei 80°C aufzeigt.

11. Faden nach Anspruch 1, wobei der Faden ein Faden der Größe 3/0 ist und einen Massenverlust von 12% nach 120 Stunden in Sorenson's Pufferlösung bei 80°C aufzeigt.

12. Verfahren zum Herstellen eines Monofilamentfadens, wie in Anspruch 1 definiert, aus einem Kunststoff eines zufälligen Copolymers, wobei das zufällige Copolymer Epsilon-Caprolactone und Glycolide umfasst, welches die folgenden Verfahrensschritte umfasst:
a. Extrudieren des Kunststoffs bei einer Extrusionstemperatur von zwischen 70°C und 215°C, um ein Monofilament bereitzustellen;
b. Dehnen des ausgehärteten Monofilaments mit einem Dehnverhältnis von zwischen 7:1 und 14:1, um ein gedehntes Monofilament bereitzustellen.

13. Verfahren nach Anspruch 12, ferner umfassend die folgenden Schritte:
a. Abkühlen des gedehnten Monofilaments bei einer Temperatur von zwischen 80°C und 180°C, um einen fertigen Faden bereitzustellen.

14. Verfahren zum Herstellen eines Monofilamentfadens, wie in Anspruch 1 definiert, aus einem Kunststoff aus einem zufälligen Copolymer, wobei das zufällige Copolymer Epsilon-Caprolactone und Glycolide umfasst, welches umfasst:
a) Extrudieren des Copolymers, um ein geschmolzenes Monofilament bereitzustellen,
b) Ablöschen des geschmolzenen Monofilaments, um ein ausgehärtetes Monofilament bereitzustellen,
c) Ziehen des ausgehärteten Monofilaments durch einen Luftofen, der auf einer Temperatur von 20°C bis 30°C gehalten wird, mit einem Zugverhältnis von 5:1 bis 10:1;
d) Ziehen des Monofilaments durch einen Luftofen, der auf einer Temperatur von 80°C bis 110°C gehalten wird, mit einem Zugverhältnis von 1,5:1 bis 1,8:1;
e) Ziehen des Monofilaments durch einen Luftofen, der auf einer Temperatur von 85°C bis 120°C gehalten wird, mit einem Zugverhältnis von 1,05:1 bis 1,06:1; und
f) Abkühlen des Monofilaments.

15. Verfahren nach Anspruch 14, wobei das zufällige Copolymer 30 Gew.-% Epsilon-Caprolactone und 70 Gew.-% Glycolide umfasst.

## Revendications

1. Suture fabriquée à partir d'un copolymère aléatoire comprenant de 68 à 75 pour cent en poids de glycolide et de 25 à 32 pour cent en poids d'epsilon-caprolactone, la suture ayant une retenue de force de deux semaines, une perte de masse d'environ 50% en 32 heures comme mesuré dans la solution tampon de Sorenson à 80°C et un module de Young de 150 kpsi à 250 kpsi et une force d'arrachage de noeud de 1,7 à 2,8 kg.

2. Suture selon la revendication 1, dans laquelle le copolymère aléatoire comprend 30 pour cent en poids d'epsilon-caprolactone et 70% en poids de glycolide.

3. Suture selon la revendication 1, où la suture est une suture de taille 3/0, et le module est 170 kpsi.

4. Suture selon la revendication 1, où la suture est une suture de taille 3/0 et la force d'arrachage de noeud est de 2,6 kg.

5. Suture selon la revendication 1, où la suture est une suture de taille 3/0 et la force de traction est de 102 kpsi.

6. Suture selon la revendication 1, où la suture est une suture de taille 3/0 et présente les caractéristiques suivantes:
| | |
|---|---|
| module | 170 kpsi |
| force d'arrachage de noeud | 2,6 kg |
| résistance à la traction | 102 kpsi. |

7. Suture selon la revendication 1, comprenant une substance médico-chirurgicalement utile.

8. Suture selon la revendication 1, où le copolymère aléatoire possède une viscosité inhérente de 1,0 à 1,8 dl/g à 30°C et une concentration de 0,25 g/dl dans HFIP.

9. Suture selon la revendication 1, où la suture est une suture de taille 3/0 et présente une perte de masse de 50% après 32 heures dans la solution tampon de Sorenson à 80°C.

10. Suture selon la revendication 1, où la suture est une suture de taille 3/0 et présente une perte de masse de 30% après 72 heures dans la solution tampon de Sorenson à 80°C.

11. Suture selon la revendication 1, où la suture est une suture de taille 3/0 et présente une perte de masse de 12% après 120 heures dans la solution de tampon de Sorenson à 80°C.

12. Procédé de fabrication d'une suture monofilament telle que définie dans la revendication 1 à partir d'une résine d'un copolymère aléatoire, le copolymère aléatoire comprenant epsilon-caprolactone et glycolide, qui comprend les opérations de:
a. extruder ladite résine à une température d'extrusion de 70°C à 215°C pour réaliser un monofilament;
b. étirer le monofilament solidifié selon un rapport d'étirage de 7:1 à 14:1 pour réaliser un monofilament étiré.

13. Procédé selon la revendication 12, comprenant en outre les étapes de:
a. recuire ledit monofilament étiré à une température de 80°C à 180°C pour réaliser une suture finie.

14. Procédé de fabrication d'une suture monofilament telle que définie dans la revendication 1, à partir d'une résine d'un copolymère aléatoire, le copolymère aléatoire comprenant epsilon-caprolactone et glycolide, qui comprend:
a) extruder le copolymère pour réaliser un monofilament fondu;
b) refroidir brusquement le monofilament fondu pour réaliser un monofilament solidifié;
c) tirer le monofilament solidifié à travers un four à air maintenu à une température de 20°C à 30°C selon un rapport de traction de 5:1 à 10:1;
d) tirer le monofilament à travers un four à air maintenu à une température de 80°C à 110°C selon un rapport d'étirage de 1,5:1 à 1,8:1;
e) tirer le monofilament à travers un four à air maintenu à une température de 85°C à 120°C selon un rapport d'étirage de 1,05:1 à 1,06:1; et
f) recuire le monofilament.

15. Procédé selon la revendication 14, où le copolymère aléatoire contient 30 pour cent en poids d'epsilon-caprolactone et 70 pour cent en poids de glycolide.
